## Europäisches Patentamt
## European Patent Office
## Office européen des brevets

(19)

(11) Veröffentlichungsnummer: **0 300 040**
**A1**

(12)

# EUROPÄISCHE PATENTANMELDUNG
Veröffentlicht nach Art. 158 Abs. 3 EPÜ

(21) Anmeldenummer: **87902190.5**

(22) Anmeldetag: **15.01.87**

Daten der zugrundeliegenden internationalen Anmeldung:

(86) Internationale Anmeldenummer:
**PCT/SU87/00003**

(87) Internationale Veröffentlichungsnummer:
**WO88/05326 (28.07.88 88/17)**

(51) Int. Cl.³: **A 63 C 11/04**

(43) Veröffentlichungstag der Anmeldung:
**25.01.89 Patentblatt 89/4**

(84) Benannte Vertragsstaaten:
**AT CH DE FR IT LI SE**

(71) Anmelder: **LOSEV, German Petrovich**
**ul. Tsvillinga, 42-16**
**Sverdlovsk, 620142(SU)**

(71) Anmelder: **KAMENSKIKH, Vasily Alexandrovich**
**ul. Belorechenskaya, 7-16**
**Sverdlovsk, 620086(SU)**

(71) Anmelder: **VAISBERG, Pavel Moishevich**
**ul. Bardina, 49-40**
**Sverdlovsk, 620147(SU)**

(84) Benannte Vertragsstaaten:

(71) Anmelder: **KHARIN, Boris Nikolaevich**
**pr. Lenina, 48-95**
**Sverdlovsk, 620075(SU)**

(72) Erfinder: **LOSEV, German Petrovich**
**ul. Tsvillinga, 42-16**
**Sverdlovsk, 620142(SU)**

(72) Erfinder: **KAMENSKIKH, Vasily Alexandrovich**
**ul. Belorechenskaya, 7-16**
**Sverdlovsk, 620086(SU)**

(72) Erfinder: **VAISBERG, Pavel Moishevich**
**ul. Bardina, 49-40**
**Sverdlovsk, 620147(SU)**

(72) Erfinder: **KHARIN, Boris Nikolaevich**
**pr. Lenina, 48-95**
**Sverdlovsk, 620075(SU)**

(74) Vertreter: **Finck, Dieter et al,**
**Patentanwälte v. Füner, Ebbinghaus, Finck Mariahilfplatz**
**2 & 3**
**D-8000 München 90(DE)**

(54) **ANORDNUNG ZUM TEST DER GLEITFLÄCHE VON SKIERN.**

(57) Einrichtung zum Prüfen der Skilauffläche, die ein Gehäuse (1) enthält, in dem eine Spiralfeder (3), die mit ihrem einen Ende am Gehäuse (1), mit ihrem anderen Ende aber auf einer in bezug auf das Gehäuse (1) drehbaren Achse (4) befestigt ist, sowie eine Lagerbuchse (7) angeordnet sind, die an dem einen Ende der Achse (4) befestigt ist. An dem außerhalb des Gehäuses (1) liegenden Ende der Lagerbuchse (7) ist eine Scheibe (8) befestigt, deren Stirnfläche (9), die zur Zusammenwirkung mit dem Schnee bzw. einer denselben nachbildenden Bedeckung bestimmt ist, aus einem Werkstoff besteht, der die zu prüfende Skilauffläche bildet. Die Vorrichtung zum Messen der relativen Drehung von Scheibe (8) und Gehäuse (1) ist in Form eines Klinkenschaltwerkes (10), dessen Zahnrad (12) auf der Achse (4) befestigt ist und das mit einem Umschalter (11) ausgestattet ist, der dazu dient, abgefederte Klinken (13, 14) mit dem Zahnrad (12) im Wechsel zum Eingriff zu bringen, einer am Gehäuse (1) angebrachten Skale (18) zum Messen der Gleitung und einem Zeiger (19) ausgeführt ist, der an dem anderen Ende der Achse (4) befestigt ist.

./...

FIG.1

## EINRICHTUNG ZUM PRÜFEN DER SKILAUFFLÄCHE

### Technisches Gebiet

Die vorliegende Erfindung bezieht sich auf Zubehör für die Skiwartung, insbesondere betrifft sie eine Einrichtung zum Prüfen der Skilauffläche.

### Zugrundeliegender Stand der Technik

Gegenwärtig ist im modernen Skisport der Erfolg eines Langlaufsportlers, Biathlonisten, alpinen Skisportlers, Skispringers, Nordisch-Kombinierten nicht nur durch physische und moralisch-kämpferische Eigenschaften sowie ihren technischen Ausbildungsgrad, sondern auch durch richtige Auswahl von Skiern bzw. eines entsprechenden Typs der aus verschiedenen Werkstoffen gefertigten Skilauffläche mit in dieselbe eingearbeiteten Rillen unterschiedlicher Form und Breite bzw. ohne dieselben sowie ferner eines entsprechenden Typs der auf die Skilauffläche aufgetragenen Schmiere bedingt.

Infolge der beispielsweise unter Temperatur- und Feuchtigkeitseinfluß veränderlichen Schneecharakteristiken, die die Reibung der Skilauffläche am Schnee bestimmen, sind zur richtigen Auswahl des Laufflächentyps und der Schmiere genaue und operative (d. h. am Starttag erfolgende) Messungen der Gleitcharakteristiken (dynamische Reibung) und Haftungscharakteristiken (statische Reibung) für eine erhebliche Anzahl der Varianten von Skilaufflächen und Skischmieren erforderlich.

Die meisten Skisportler wählen ihre Skier und die Skischmiere durch mehrfaches Ausprobieren einer beschränkten Zahl von möglichen Varianten von Skilaufflächen und Skischmieren. Dies ist ein langwieriger und arbeitsintensiver Prozeß, der trotzdem keine ausreichend genauen Resultate liefert. Daher stützen sich die Sportler und ihre Trainer bei der Auswahl von Skiern, eines jeweiligen Typs von Skilauffläche und Skischmiere meist auf eigene Erfahrungen und Intuition.

Die bekannten Einrichtungen zum Prüfen der Skilauffläche mit der auf dieselbe aufgetragenen Schmiere bzw. ohne dieselbe erlauben es wegen ihrer konstruktiven Besonderheiten ebenfalls nicht, den erforderlichen Typ von Skiern, Skilauffläche und Skischmiere sicher und operativ zu wählen.

Bekannt ist eine Einrichtung zur Auswahl der Skischmiere (UdSSR-Urheberschein Nr. 188340, IPK A 63 C 11/04, veröffentlicht im Bulletin "Entdeckungen, Erfindungen, Geschmacksmuster, Warenzeichen" Nr. 21, 1966), die ein Skimodell mit einer auf die Lauffläche desselben aufgetragenen Schmiere sowie eine Stoßvorrichtung zum Ausrollen des Skimodells auf dem Schnee enthält, die in Gestalt eines abgefederten Stößels ausgeführt ist. Die Einrichtung ist zur Ermittlung von Gleiteigenschaften verschiedener Schmieren bestimmt und beruht auf dem Messen der Ausrollänge des Skimodells bei dessen fortschreitender Bewegung.

Die beschriebene Einrichtung besitzt geringe Schnellwirkung, weil eine lange Zeit für das Ausrollen des Skimodells, die Ablesung der Ausrollänge und Rückführung des Skimoddels in die Ausgangslage erforderlich ist. Diese Einrichtung ist ferner durch niedrige Genauigkeit der Messung der Gleiteigenschaften infolge starker Beeinflussung durch statische Reibung (bei einer in der Nullnähe liegenden Geschwindigkeit) und Unebenheiten der Schneeoberfläche gekennzeichnet, auf der die Messungen erfolgen, und besitzt begrenzte funktionale Möglichkeiten, weil die Wahl der Skischmiere lediglich auf der Messung des Gleitungsparameters beruht.

Bekannt ist eine Einrichtung zum Prüfen der Skilauffläche (UdSSR-Urheberschein Nr. 787051. IPK A 63 C 11/04, veröffentlicht im Bulletin Entdeckungen,Erfindungen, Geschmacksmuster, Warenzeichen " Nr. 46, 1980),die ein Gehäuse enthält, in dem eine Spiralfeder, die mit ihrem einen Ende am Gehäuse, mit ihrem anderen Ende aber auf der Achse befestigt ist, eine Lagerbuchse, an deren außerhalb des Gehäuses liegendem Ende eine Scheibe befestigt ist, deren Stirnfläche, die zur Zusammenwirkung mit dem Schnee bzw. mit einer denselben nachbildenden Bedeckung bestimmt ist, aus einem Werkstoff besteht, der die zu prüfende Skilauffläche bildet, sowie eine Vorrichtung zum Messen der relativen Drehung von Scheibe und Gehäuse angeordnet sind.

Die Vorrichtung zum Messen der relativen Drehung von Scheibe und Gehäuse enthält einen Teilkreis mit einer Skale und einem Griff sowie eine Freilaufkupplung zur Feststellung des Betrags der relativen Drehung, die die Haftung der zu prüfenden Skilauffläche mit der auf dieselbe aufgetragenen Schmiere an dem Schnee bzw. einer denselben nachbildenden Bedeckung charakterisiert.

Nach dem Aufstellen der Einrichtung auf Schnee beginnt man, den Teilkreisgriff zu drehen. Die mit dem Teilkreis und der Lagerbuchse verbundene Spiralfeder fängt an, sich zu verdrehen und erzeugt ein zunehmendes Drehmoment an der Lagerbuchse. Die Verdrehung dauert so lange, bis dieses Drehmoment das Widerstandsmoment übersteigen wird, das durch die Kraft der Haftung der zu prüfenden Skilauffläche am Schnee entsteht. Die Scheibe dreht sich gemeinsam mit der Lagerbuchse nach derselben Seite hin wie der Teilkreis, wobei der Augenblick des Drehbeginns visuell festgestellt wird und man mit dem Drehen des Griffes demgemäß aufhört, während der Betrag des Verdrehungswinkels an der Skale abgelesen wird. Die Feststellung des Augenblicks des Drehbeginns erfolgt dank dem durch die Kugel der Freilaufkupplung erzeugten Festklemmen des Spaltes zwischen dem Teilkreis und dem Gehäuse bei Änderung der relativen Drehrichtung. Auf Grund der gewonnenen Meßergebnisse beim Messen der Haftung, das mit verschiedenen Scheiben durchgeführt wird, deren Stirnfläche mit verschiedenen Schmieren bedeckt ist, wählt man die für die jeweiligen Verhältnisse optimale Schmiere.

Die bekannte Einrichtung zum Prüfen der Skilauffläche besitzt begrenzte funktionale Möglichkeiten, weil die beschriebene konstruktive Ausführung der Vorrichtung zum Messen der relativen Drehung von Scheibe und Gehäuse nur das Messen des Haftungsparameters gestattet.

Die Meßgenauigkeit ist wegen der bedeutenden Unempfindlichkeitszone der Freilaufkupplung nicht hoch, die den Augenblick des Drehbeginns der Scheibe nicht genau genug fixiert. Das Drehen des Gehäuses mit Hilfe eines da-

- 4 -

ran befestigten Griffs führt zum "schädlichen" Ändern des durch die Einrichtung auf den Schnee ausgeübten Druckes und zu einer Verschiebung des Gehäuses, was einen verfrühten bzw. verspäteten Scheibendrehbeginn bedingt. Die Einrichtung ist derart aufgebaut, daß in ihr keine Möglichkeit zum Vermindern von bei Änderungen entstehenden zufälligen Fehlern vorgesehen ist.

Die bekannte Einrichtung ist ferner durch eine unzureichende Schnellwirkung gekennzeichnet und ist nicht handlich, weil bei einer jeden Messung auf der Teilkreisskale eine "Null" eingestellt und die Einrichtung zum Ablesen des Meßergebnisses von der Schneeoberfläche abgehoben werden muß, da die Teilkreisskale an der Seitenfläche des Gehäuses angebracht ist.

Da die in der bekannten Einrichtung verwendete Lagerbuchse einen größeren Durchmesser besitzt und die Feststellung der Scheibe mittels abgefederten Feststellern erfolgt, sind beträchtliche Schiefstellungen der Scheibenachse und das Festklemmen der abgefederten Feststeller möglich, was die für das Auswechseln der Scheiben benötigte Zeit verlängert.

Offenbarung der Erfindung

Der Erfindung liegt die Aufgabe zugrunde, eine Einrichtung zum Prüfen der Skilauffläche mit einer solchen konstruktiven Ausführung der Vorrichtung zum Messen der relativen Drehung von Scheibe und Gehäuse zu schaffen, die es erlauben würde, das Gleiten der zu prüfenden Lauffläche über Schnee bzw. eine denselben nachbildende Bedeckung zu ermitteln.

Die gestellte Aufgabe ist dadurch gelöst, daß in der Einrichtung zum Prüfen der Skilauffläche, die ein Gehäuse enthält, in dem eine Spiralfeder, die mit ihrem einen Ende am Gehäuse, mit ihrem anderen Ende aber auf einer Achse befestigt ist, eine Lagerbuchse, an dessen außerhalb des Gehäuses liegendem Ende eine Scheibe befestigt ist, deren Stirnfläche, die zur Zusammenwirkung mit dem Schnee bzw. einer denselben nachbildenden Bedeckung bestimmt ist, aus einem Werkstoff besteht, der die zu prüfende Skilauffläche bildet, sowie eine Vorrichtung zum Messen der relativen Drehung von Scheibe und Gehäuse angeordnet sind, erfindungsgemäß die Achse drehbar angebracht und an deren einem Ende die Lagerbuchse befestigt ist, während die Vorrichtung zum Messen der relativen Drehung von Scheibe und Gehäuse in Form eines Klinkenschaltwerks, dessen Zahnrad auf der Achse befestigt ist und das mit einem Umschalter ausgestattet ist, der die abgefederten Klinken mit dem Zahnrad im Wechsel zum Eingriff bringt, einer am Gehäuse angebrachten Skale zum Gleitungsmessen und einem an dem anderen Achsenende befestigten Zeiger ausgeführt ist.

Zweckmäßigerweise enthält die Einrichtung zum Prüfen der Skilauffläche ein Stativ, das in Gestalt einer ersten Grundplatte zum Aufsetzen derselben auf dem Schnee bzw. einer denselben nachbildenden Bedeckung, einer zweiten Grundplatte mit Bohrung und eines Griffringes ausgeführt ist, der in der erwähnten Bohrung relativ zur Bohrungsachse drehbar untergebracht ist, während das Gehäuse im Ring axial verschiebbar angeordnet ist, wobei die Vorrichtung zum Messen der relativen Drehung von Scheibe und Gehäuse eine am Gehäuse angebrachte Skale zum Ablesen der Ergebnisse der Haftungsmessung enthält.

Es ist ferner zweckmäßig, daß die Einrichtung zum Prüfen der Skilauffläche einen elastischen Ring enthält und an der inneren Seitenfläche der Lagerbuchse eine Rille ausgeführt ist, in der dieser elastische Ring aufgenommen ist, wobei an der Scheibe ein zylindrischer Vorsprung mit einer an dessen Stirnfläche ausgeführten Nut und einer Rille zum Festhalten der Scheibe durch den erwähnten elastischen Ring ausgeführt ist, während am entsprechenden Achsenende ein Vorsprung ausgebildet ist, der sich in der Scheibennut befindet.

Es empfiehlt sich, daß das Gehäuse mit einem Halter zum Anbringen eines abnehmbaren Steuerorgans zur Betätigung des Umschalters des Klinkenschaltwerks ausgestattet ist.

Vorzugsweise soll die Einrichtung zum Prüfen der Skilauffläche einen Ring enthalten, der am Gehäuse drehbar angeordnet ist, während die Vorrichtung zum Messen der relativen Drehung von Scheibe und Gehäuse zusätzlich eine an diesem Ring angebrachte Skale zum Mitteln von Meßergebnissen enthalten soll.

Die erfindungsgemäße Einrichtung zum Prüfen der Skilauffläche gestattet es, zwei Parameter zu messen, die zur Wahl der erforderlichen Skischmiere bzw. des Skityps unumgänglich sind, und zwar die Gleitung und die Haftung. Das Messen dieser Parameter setzt keine speziellen Meßplätze voraus, weil die Messungen unmittelbar in der Skispur vor dem Start durchgeführt werden können. Das Messen der Gleitung und der Haftung erfolgt mit einer hohen Genauigkeit dank dem erfindungsgemäß vorgesehenen Klinkenschaltwerk mit einem Umschalter, der eine präzise Fixierung des Verdrehungswinkels der Spiralfeder und die Stillsetzung der Scheibe gewährleistet. Die Betätigung des Umschalters von einem abnehmbaren Steuerorgan setzt die mit der Handsteuerung zusammenhängenden Fehler beträchtlich herab, und die Einführung einer zum Mitteln der Meßergebnisse bestimmten Skale sichert die Vermeidung von zufälligen Fehlern beim Messen.

Die Einrichtung ist einfach zu handhaben, die Abmessungen der beweglichen Teile sind in ihr wesentlich verringert, was die Drehgeschwindigkeit der Scheibe zu steigern und somit also die Schnellwirkung zu erhöhen erlaubt. Die Einführung eines Skale-Zeiger-Systems sowie die Anbringung der Skalen am Gehäusedeckel vereinfacht das Ablesen der Meßergebnisse.

Insgesamt erlaubt das alles, die Operativität der mit der erfindungsgemäßen Einrichtung vorgenommenen Messungen zu erhöhen und die Prüfdauer von 10 Arten von Skischmieren bzw. 20 Arten von Skiparaffinen bis auf 30 min herabzusetzen.

Kurze Beschreibung der Zeichnungen

Im folgenden wird die Erfindung in der Beschreibung konkreter Ausführungsbeispiele derselben sowie durch beigefügte Zeichnungen erläutert, in denen es zeigt:

Fig. 1 die Gesamtansicht der Einrichtung zum Prüfen der Skilauffläche (im Längsschnitt), gemäß der Erfindung;

Fig. 2 einen Schnitt nach Linie II-II der Fig. 1,
gemäß der Erfindung;

Fig. 3 die Gesamtansicht der Einrichtung zum Prüfen
der Skilauffläche mit einem Stativ (teilweise ausgebrochen), gemäß der Erfindung;

Fig. 4 eine Ansicht in Pfeilrichtung A von Fig.3,
gemäß der Erfindung.

Beste Ausführungsform der Erfindung

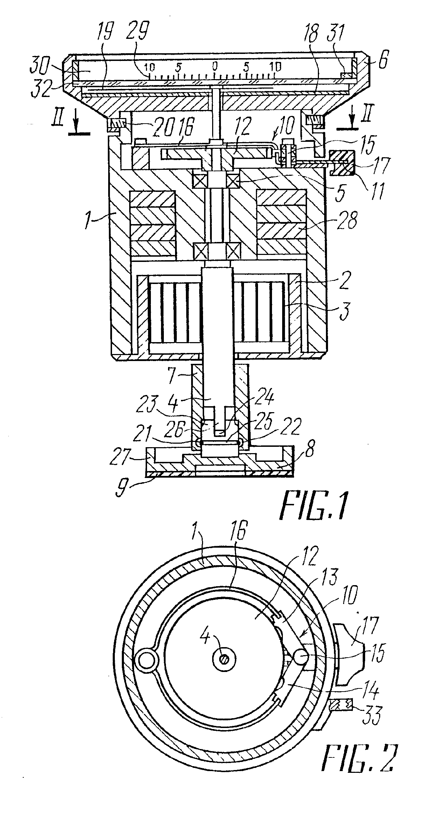

Die Einrichtung zum Prüfen der Skilauffläche enthält
ein Gehäuse 1 (Fig. 1), in dem in der vorliegenden Ausführungsform der Erfindung ein Zylinder 2 starr befestigt
und eine Spiralfeder 3 angebracht ist, die mit ihrem einen Ende am Zylinder 2, mit ihrem anderen Ende aber auf
einer Achse 4 befestigt ist, die in Lagern 5 gleichachsig
mit dem Zylinder 1 in bezug auf das Gehäuse 1 drehbar aufgenommen ist. Das Gehäuse 1 ist mit einem Deckel 6 versehen, und auf der Achse 4 ist eine Lagerbuchse 7 befestigt,
deren eines Ende sich außerhalb des Gehäuses 1 befindet
(in der vorliegenden Ausführungsform liegt die gesamte
Lagerbuchse 7 außerhalb des Gehäuses 1). An diesem Ende
der Lagerbuchse 7 ist eine Scheibe 8 angebracht, deren
Stirnfläche 9, die zur Zusammenwirkung mit dem Schnee bzw.
mit einer denselben nachbildenden Bedeckung bestimmt ist,
aus einem beispielsweise einem Plast, besteht, der die
zu prüfende Skilauffläche bildet, auf die eine Skischmiere bzw. eine Kombination von solchen Skischmieren, ein
Skiparaffin bzw. eine Kombination von Skiparaffinen aufgetragen werden können. Außerdem können in die zu prüfende Skilauffläche Längsrillen unterschiedlicher Konfiguration eingearbeitet werden.

Die Einrichtung enthält ferner eine Vorrichtung zum
Messen der relativen Drehung von Scheibe 8 und Gehäuse 1,
die in Form eines Klinkenschaltwerks 10 ausgeführt ist,
das mit einem Umschalter 11 ausgestattet ist. Ein Zahnrad 12 des Klinkenschaltwerks 10 ist auf der Achse 4 be-

- 8 -

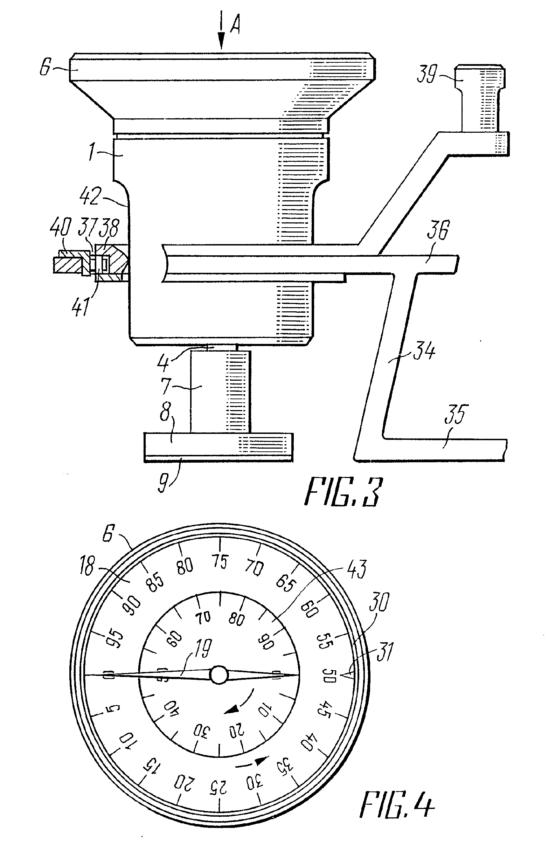

festigt, der Umschalter 11 dient dazu, Klinken 13 (Fig.2), 14, die auf einer Achse 15 angebracht und an das Zahnrad 12 durch eine Feder 16 angedrückt sind, mit diesem Zahnrad 12 im Wechsel in Eingriff zu bringen. In der vorliegenden Ausführungsform stellt der Umschalter 11 (Fig.1) einen Stab dar und besitzt zwei Endstellungen und eine Mittelstellung (in Fig. 2 entspricht die Lage der Klinken 13, 14 der Mittelstellung des Umschalters 11). Zum bequemen Bedienen ist der Umschalter 11 mit einem Griff 17 (Fig. 1,2) versehen, der am Gehäuse 1 angebracht ist. Die Vorrichtung zum Messen der relativen Drehung von Scheibe 8 und Gehäuse 1 schließt auch eine am Deckel 6 des Gehäuse 1 angebrachte Skale 18 (Fig. 1) zum Bestimmen der Gleitung, sowie einen Zeiger 19 ein, der an dem anderen Ende der Achse 4 befestigt ist. Der Deckel 6 des Gehäuses 1 ist in bezug auf dasselbe drehbar angeordnet, um die "Null" der Skale 18 mit dem Zeiger 19 bei fehlendem Drehmoment in Übereinstimmung zu bringen, das sonst durch die Spiralfeder 3 erzeugt wird. Die genannte Lage der Skale 18 wird mittels Sicherungsschrauben 20 festgestellt.

Zum raschen und sicheren Anbringen der Scheibe 8 auf der Lagerbuchse 7 ist in der Einrichtung ein weiterer elastischer Ring 21 vorgesehen, wobei an der inneren Seitenfläche der Lagerbuchse 7 eine Rille 22 eingearbeitet ist, in der dieser Ring 21 untergebracht ist, und an der Scheibe 8 ist ein zylindrischer Vorsprung 23 mit einer Nut 24 an ihrer Stirnfläche und einer Rille 25 zum Festhalten der Scheibe 8 durch den elastischen Ring 21 ausgebildet. Am entsprechenden Ende der Achse 4 ist ein Vorsprung 26 ausgeführt, der in der Nut 24 der Scheibe 8 aufgenommen ist. Zum bequemen Handhaben ist die Scheibe 8 mit einem Hemd 27 mit Hieb versehen, der das Durchgleiten derselben beim Anbringen und Abnehmen von der Lagerbuchse 7 ausschließt.

In der vorliegenden Ausführungsform der Einrichtung ist die Möglichkeit vorgesehen, den spezifischen Druck

0300040

der Scheibe 8 auf den Schnee bzw. eine denselben nachbildende Bedeckung je nach Gewicht des Sportlers und der Beschaffenheit der Lauffläche seiner Skier zu ändern. Hierzu ist im Gehäuse 1 eine erforderliche Anzahl von Wechselgewichten 28 untergebracht.

Zum Mitteln der Meßergebnisse enthält die Vorrichtung zum Messen der relativen Drehung von Scheibe 8 und Gehäuse 1 eine Skale 29 zum Mitteln der Meßergebnisse, die in vereinbarten Meßeinheiten graduiert ist und auf einem elastischen Ring 30 mit einem Anzeiger 31 angebracht ist, der an der Seitenfläche des Deckels 6 drehbar angeordnet ist. Ein im Deckel 6 unter der Skale 18 vorhandenes Glas 32 schützt die Skale 18 und den Zeiger 19 vor Brüchen und Verunreinigung.

Beim Messen von Gleitungsparametern ist das Gehäuse 1 zur Betätigung des Umschalters 11 des Klinkenschaltwerks 10 von einem abnehmbaren Steuerorgan, beispielsweise einem Seilauslöser einer Fotokamera bzw. einem automatischen Auslöser (in der Zeichnung nicht abgebildet), mit einem Halter 33 (Fig. 2) versehen. Dabei ist die Übertragung einer "schädlichen" Kraft (zusätzlicher Druck und Gehäuseverschiebung) auf die Einrichtung bei manuellem Bedienen des Umschalters 11 unterbunden.

Zum Messen der Haftung enthält die Einrichtung zum Prüfen der Skilauffläche ein Stativ 34 (Fig. 3), das in Gestalt einer ersten Grundplatte 35 zum Aufsetzen derselben auf Schnee bzw. einer denselben nachbildenden Bedeckung, einer zweiten Grundplatte 36 mit einer Bohrung 37, einem Ring 38 mit Griff 39 ausgeführt ist, der in dieser Bohrung 37 mit Hilfe eines Kragstücks 40 in Lagern 41 in bezug auf die Achse der Bohrung 37 drehbar aufgenommen ist. Am Gehäuse 1 sind Fasen 42 ausgeführt, die die Drehung des Gehäuses 1 in bezug auf den Ring 38 verhindern und die Möglichkeit einer axialen Verschiebung desselben gewährleisten.

Die Vorrichtung zum Messen der relativen Drehung von Scheibe 8 und Gehäuse 1 enthält eine Skale 43 (Fig.4) zum Messen der Haftung, die am Deckel 6 des Gehäuses 1 konzentrisch zur Skale 18 zum Messen der Gleitung angebracht ist. Zum bequemeren Ablesen der Gleitungs- und Haftungswerte ist die "0" der Skale 18 in bezug auf die "0" der Skale 43 um 180° verschoben, und zum genaueren Ablesen des Gleitungskennwertes liegt die Skale 18 am Umfang eines größeren Kreises, wobei zum Ablesen der Haftung das kleinere Ende des Zeigers 19 dient.

Jede Skale 18, 43 ist in vereinbarten Meßeinheiten graduiert und weist je 100 Teilungen auf. Die Zahl der Teilungen ist so gewählt, daß eine höhere Meßgenauigkeit und eine bequemere Handhabung der Einrichtung erzielbar wären, wobei man den Teilungswert nicht zu wissen braucht, weil eben die vergleichende Bewertung von Gleitung und Haftung für die zu prüfenden Skilaufflächen von Bedeutung ist.

Die erfindungsgemäße Einrichtung ermöglicht es, Prüfungen von Skilaufflächen in zwei Regimes durchzuführen: im Regime der dynamischen Reibung (Gleiten) und im Regime der statischen Reibung (Haftung).

Vor dem Meßbeginn bringt man die Einrichtung in die Ausgangsstellung, wozu man auf die Lagerbuchse 7 (Fig.1) die Scheibe 8 mit z.B. auf deren Stirnfläche 9 aufgetragener Schmiere aufsetzt. Die Scheibe 8 wird in die Lagerbuchse 7 mit seinem zylindrischen Vorsprung 23 eingesetzt, wonach durch Drehen um die Achse 4 der Vorsprung 26 der Achse 4 mit der Nut 24 des zylindrischen Vorsprungs 23 in Übereinstimmung gebracht wird. Hierbei wird eine freie Drehung der Scheibe 8 in bezug auf die Achse 4 ausgeschlossen. Danach verschiebt man den zylindrischen Vorsprung 23 ins Innere der Lagerbuchse 7 bis zum vollständigen Zusammenfallen des elastischen Ringes 21 mit der Rille 21, der das Festhalten der Scheibe 8 in der Lagerbuchse 7 gewährleistet.

Zum Messen der Gleitung führt man den Umschalter 11 im Uhrzeigersinn in eine Endstellung (Stellung 1) mittels des Griffes 17 über, wobei der Umschalter 11 die Klinke 13 (Fig. 2) außer Eingriff mit dem Zahnrad 12

- 11 -

und die Klinke 14 in Eingriff mit demselben bringt, die an das Zahnrad 12 durch die Feder 16 angedrückt wird. Diese Stellung des Umschalters 11 gestattet es, die Verdrehung der Spiralfeder 3 (Fig. 1) einzuleiten. Durch Drehen der Scheibe 8 im Uhrzeigersinn in bezug auf das Gehäuse 1 verdreht man die Spiralfeder 3 um einen bestimmten Winkel, beispielsweise um 360°, was 100 Teilungen der Skale 18 (Fig. 4) zum Messen der Gleitung entspricht. Hierbei hält das Klinkenschaltwerk 10 (Fig. 1) den Zeiger 19, die Scheibe 8 und die Spiralfeder 3 in der eingestellten Lage fest. Danach stellt man die Einrichtung auf Schnee bzw. einer denselben nachbildenden Bedeckung mit der Stirnfläche 9 der Scheibe 8 auf und führt von Hand den Umschalter 11 entgegen dem Uhrzeigersinn in eine Endstellung (Stellung II) über, bei der der Umschalter 11 die Klinke 14 (Fig. 2) außer Eingriff mit dem Zahnrad 12 und die Klinke 12 in Eingriff mit demselben bringt. Hierbei fängt die Achse 4 (Fig.1) mit der Scheibe 8 und dem Zeiger 9 unter der Wirkung des durch die Spiralfeder 3 erzeugten Momentes sowie des Momentes, das durch die Gleitreibungskraft erzeugt wird und zwischen der Stirnfläche 9 der Scheibe 8, d. h. also zwischen der zu prüfenden Skilauffläche, und dem Schnee entsteht, sich aufzudrehen an. Im Augenblick des Richtungswechsels der Winkelgeschwindigkeit der Achse 4 erfolgt deren Stillsetzung gemeinsam mit der Scheibe 8, wobei der Zeiger gegenüber einer bestimmten Teilung der Skale 18 (Fig. 4) zum Messen der Gleitung stehenbleibt, beispielsweise gegenüber der Teilung "60", die dem Gleitungsparameter von 60 vereinbarten Einheiten entspricht. Die Anzeigen werden abgelesen und in eine Tabelle eingetragen bzw. durch Einstellen des Anzeigers 31 gegenüber dem größeren Ende des Zeigers 19 fixiert. Die Drehung der Achse 4 (Fig. 2) mit der Scheibe 8 in der entgegengesetzten Richtung wird durch die abgefederte Klinke 13 verhindert. Der Einsatz eines Klinkenschaltwerks 10 (Fig. 1) gestattet es, den Augenblick der Beendigung der Drehung der Achse 4 und der Scheibe 8 mit hoher Genauigkeit zu fixieren sowie die

- 12 -

Verdrehung der Spiralfeder 3 um einen bestimmten Winkel mit nachfolgender Verwertung der Energie dieser Feder 3 zum Drehen der Scheibe 8 vorzunehmen.

Zur Erhöhung der Meßgenauigkeit kann eine Messung mit ein und derselben Scheibe 8 mehrere Male unter Mittelung der Meßergebnisse wiederholt werden. Das zweite Meßergebnis wird ebenfalls abgelesen und in die Tabelle eingetragen, während mit Hilfe der Skale 29 zum Mitteln der Meßergebnisse dieses Resultat mit dem vorhergehenden Resultat unter nachfolgender Bestimmung des mittleren Ergebnisses summiert werden kann. Hierzu wird der Anzeiger 31 (Fig. 4) an das größere Ende des Zeigers 19 um einen halben Anzeigewert gemäß der Skale 29 (Fig. 1) übergeführt. Nach einer dritten, vierten usw. Messung wird der Anzeiger 31 in derselben Richtung, wie bereits beschrieben, um 1/3, 1/4 usw. des Anzeigewertes gemäß der Skale 29 verschoben. Der Anzeiger 31 fixiert stets des mittlere Resultat aus sämtlichen vorgenommenen Messungen.

Braucht man eine genauere Messung der Gleitungsparameter, so nimmt man die Überführung des Umschalters 11 (Fig. 1, 2) in eine Endstellung (Stellung II) mit Hilfe irgendeines abnehmbaren Steuerorgans vor, das im Halter 33 (Fig. 2) befestigt ist und die Übertragung der "schädlichen" Kraft von der Hand der die Prüfung vornehmenden Person ausschließt, welche Kraft zur Erzeugung eines zusätzlichen Druckes auf die Einrichtung bzw. zur Verschiebung derselben vom gewählten Prüfungsort führt. Die Anwendung eines abnehmbaren Steuerorgans zur Betätigung des Umschalters 11 (Fig. 11) gestattet es, die Zahl der durchzuführenden Messungen zu vermindern und hierdurch die Operativität zu erhöhen, was auch durch das eingeführte System Skale 18 - Zeiger 19 gefördert wird, wobei dessen Lage am Deckel 6 des Gehäuses 1 zum Ablesen von Meßergebnissen sehr bequem ist.

Entsprechend den Ergebnissen der Gleitungsparametermessungen wählt man einen für konkrete Bedingungen optimalen Typ der Skilauffläche mit auf dieselbe aufgetragener Skischmiere (Paraffin) bzw. ohne dieselbe. Für sol-

che Sportarten wie Skispringen, Alpiner Skisport, Skilanglauf (bei zugelassener Anwendung von Schlittschuhschritt) reicht es bereits aus, nur den einen Parameter - die Gleitung - zu bestimmen, um den Wettbewerbserfolg zu sichern, während man in den anderen Sportarten - Nordische Kombination, Skilanglauf außer Gleitung auch der andere Parameter - die Haftung- bekannt sein muß.

Das Messen der Haftung erfolgt nach dem Einsetzen des Gehäuses 1 in den Ring 38 (Fig. 3), derart, daß die mit Skischmiere bedeckte Stirnfläche 9 der Scheibe 8 mit dem Schnee bzw. der denselben nachbildenden Bedeckung kontaktiert. Der Umschalter 11 (Fig. 1) wird in die Endstellung "I" übergeführt, wonach man durch Drehen des Griffes 39 (Fig. 3) den Ring 38 mit dem in diesen eingesetzten Gehäuse 1 gegen den Uhrzeigersinn dreht. Hierbei fängt die Spiralfeder 3 (Fig. 1) sich zu verdrehen an, und es entsteht ein proportional dem Verdrehungswinkel zunehmendes Drehmoment an der Scheibe 8. Gleichzeitig mit dem Drehen des Gehäuses 1 wird auch die Skale 43 (Fig.4) zum Messen der Haftung in Drehung versetzt, während der mit der Scheibe 8 (Fig. 1) verbundene Zeiger 19 unbeweglich bleibt. Das Drehen des Griffes 39 (Fig. 3) und die demzufolge stattfindende Verdrehung der Spiralfeder 3 (Fig. 1) setzt man so lange fort, bis sich der Zeiger 19 zu drehen anfängt. Das bedeutet, daß das von der Spiralfeder 3 erzeugte Drehmoment das Widerstandsmoment gegen die Drehung, das durch die Haftungskraft zwischen der Stirnfläche 9 der Scheibe 8 und dem Schnee bzw. der denselben nachbildenden Bedeckung zustande kommt, überstiegen hat. Im Augenblick des Drehbeginns des Zeigers 19 sowie nach Aufhören der Drehung desselben mittels Griffes 39 (Fig. 3) ist der Zeiger 19 (Fig. 4) in bezug auf eine bestimmte Teilung der Skale 43 zum Messen der Haftung fixiert, auf die das kleinere Ende des Zeigers 19 weist. Die Drehung der Achse 4 (Fig. 1) mit der Scheibe 8 unter der Wirkung der verdrehten Feder nach der entgegengesetzten Richtung ist durch die abgefederte Klinke 14 (Fig. 2) verhindert, die mit dem Zahnrad 12 im Eingriff steht.

- 14 -

Die Anzeige wird abgelesen und in eine Tabelle eingetragen bzw. mit Hilfe des Anzeigers 31 (Fig. 1) "gespeichert". Die Messungen können mehrere Male unter Mittelung von Meßergebnissen ähnlich wie vorbeschrieben wiederholt werden.

Nach den Messungen mit einer Scheibe 8 (mit einem Typ der Skischmiere) geht man zu Messungen mit einer anderen Scheibe 8 (einem anderen Typ der Skischmiere) über.

In der Einrichtung ist die Möglichkeit vorgesehen, den spezifischen Druck je nach Gewicht des Sportlers bzw. je nach der Beschaffenheit der Lauffläche seiner Skier zu ändern. Dies wird durch Entfernen bzw. Hinzufügen von Gewichten 28 erreicht.

Es ist zu vermerken, daß man das Messen der Gleitung auch mit dem im Stativ 34 (Fig. 3) angeordneten Gehäuse 1 durchführen kann, während zur Erhöhung der Meßgenauigkeit zweckmäßigerweise ein abnehmbares Steuerorgan zur Betätigung des Umschalters 11 (Fig. 1) ähnlich wie vorstehend beschrieben angewendet werden kann.

Also gestattet es die erfindungsgemäße Einrichtung zum Prüfen der Skilauffläche, die Gleitungs- und Haftungsparameter genau und operativ zu bestimmen, und mit deren Hilfe eine für die jeweiligen Bedingungen optimale Skischmiere bzw. einen optimalen Skityp zu wählen.

Gewerbliche Verwertbarkeit

Die vorliegende Erfindung kann im modernen Skisport (im Skilanglauf, Skispringen, Biathlon, im alpinen Skisport, in der Nordischen Kombination) zur Bestimmung von Gleit- und Haftungseigenschaften verschiedener Skilaufflächen, darunter auch mit auf dieselben aufgetragenen unterschiedlichen Schmieren, bzw. der auf irgendeine Weise mechanisch bearbeiteten Skilaufflächen angewendet werden.

- 15 -

PATENTANSPRÜCHE

1. Einrichtung zum Prüfen der Skilauffläche, die ein Gehäuse (1) enthält, in dem eine Spiralfeder (3), die mit ihrem einen Ende am Gehäuse (1), mit ihrem anderen Ende auf einer Achse (4) befestigt ist, eine Lagerbuchse (7), an deren außerhalb des Gehäuses (1) liegendem Ende eine Scheibe (8) befestigt ist, deren Stirnfläche (9), die zur Zusammenwirkung mit dem Schnee bzw. einer denselben nachbildenden Bedeckung bestimmt ist, aus einem Werkstoff besteht, der die zu prüfende Skilauffläche bildet, sowie eine Vorrichtung zum Messen der relativen Drehung von Scheibe (8) und Gehäuse (1) angeordnet sind, dadurch g e k e n n z e i c h n e t , daß die Achse (4) drehbar gelagert und an deren einem Ende die Lagerbuchse befestigt ist, während die Vorrichtung zum Messen der relativen Drehung von Scheibe (8) und Gehäus (1) in Form eines Klinkenschaltwerkes 10 dessen Zahnrad (12) auf der Achse (4) befestigt ist und das mit einem Umschalter (11) ausgestattet ist, der dazu dient, abgefederte Klinken (13, 14) mit dem Zahnrad (12) im Wechsel zum Eingriff zu bringen, einer am Gehäuse (1) angebrachten Skale (18) zum Messen der Gleitung und einem Zeiger (19) ausgeführt ist, der an dem anderen Ende der Achse (4) befestigt ist.

2. Einrichtung zum Prüfen der Skilauffläche nach Anspruch 1, dadurch g e k e n n z e i c h n e t , daß sie ein Stativ (34) enthält, das in Gestalt einer ersten Grundplatte (35) zum Aufsetzen derselben auf Schnee bzw. einer denselben nachbildenden Bedeckung, einer zweiten Grundplatte (36) mit Bohrung (37) und einem Ring (38) mit Griff (39) ausgeführt ist, der in dieser Bohrung (37) in bezug auf die Achse derselben drehbar untergebracht ist, während das Gehäuse (1) im Ring (38) axial verschiebbar angeordnet ist, wobei die Vorrichtung zum Messen der relativen Drehung von Scheibe (8) und Gehäuse (1) eine am Gehäuse (1) angebrachte Skale (43) zum Messen der Haftung enthält.

3. Einrichtung zum Prüfen der Skilauffläche nach Ansprüchen 1 bzw. 2, dadurch g e k e n n z e i c h n e t ,

daß sie einen elastischen Ring (21) enthält und an der inneren Seitenfläche der Langerbuchse (7) eine Rille (20) ausgeführt ist, in der dieser elastische Ring (21) aufgenommen ist, wobei an der Scheibe (8) ein zylindrischer Vorsprung (23) mit einer Nut (24) an deren Stirnfläche und einer Rille (25) zum Festhalten der Scheibe (8) durch den elastischen Ring (21) ausgeführt ist, während am entsprechenden Ende der Achse (4) ein Vorsprung (26) ausgebildet ist, der sich in der Nut (24) der Scheibe (8) befindet.

4. Einrichtung zum Prüfen der Skilauffläche nach einem beliebigen von den Patentansprüchen 1 bis 3, dadurch g e k e n n z e i c h n e t, daß das Gehäuse (1) mit einem Halter (33) zum Anbringen eines abnehmbaren Steuerorgans zur Betätigung des Umschalters (11) des Klinkenschaltwerkes (10) ausgestattet ist.

5. Einrichtung zum Prüfen der Skilauffläche nach einem beliebigen von den Patentansprüchen 1 bis 4, dadurch g e k e n n z e i c h n e t, daß sie einen Ring (30) enthält, der am Gehäuse (1) drehbar angeordnet ist, während die Vorrichtung zum Messen der relativen Drehung von Scheibe (8) und Gehäuse (1) zusätzlich eine an diesem Ring (30) angebrachte Skale (29) zum Mitteln von Meßergebnissen enthält.

*FIG.1*

*FIG.2*

0300040

2

A

6

1

42

40 37 38

41

4

7

8

9

36

34

35

39

**FIG.3**

6

18

80 75 70

85

90

95

43

65

60

30

55

31

70 80

60

19

90

50

40

45

5

10

20

10

30

40

15

35

20

25 30

**FIG.4**

# INTERNATIONAL SEARCH REPORT  0300040

International Application No PCT/SU 87/00003

## I. CLASSIFICATION OF SUBJECT MATTER (if several classification symbols apply, indicate all) [6]

According to International Patent Classification (IPC) or to both National Classification and IPC

IPC [4]: A63C 11/04

## II. FIELDS SEARCHED

### Minimum Documentation Searched [7]

| Classification System | Classification Symbols |
|---|---|
| IPC [4]: | A63C 11/04 |

Documentation Searched other than Minimum Documentation
to the Extent that such Documents are Included in the Fields Searched [8]

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| A | SU, A1, 188340 (N.I. Kuzmin) 07 December 1966 (07.12.66) see the claims (cited in the description) | 1-5 |
| A | SU, A1, 787051 (G.P. Losev et al.) 25 December 1980 (25.12.80) see the claims (cited in the description) | 1-5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 21 September 1987 (21.09.87) | 14 October 1987 (14.10.87) |
| International Searching Authority | Signature of Authorized Officer |
| ISA/SU | |

Form PCT/ISA/210 (second sheet) (January 1985)